# EUROPEAN PATENT SPECIFICATION

(11) **EP 1 842 922 B1**
(45) Date of publication and mention of the grant of the patent: **25.05.2011**
(21) Application number: 06125592.3
(22) Date of filing: 07.12.2006
(51) Int. Cl.: C12Q 1/68

(54) **Method of increasing specificity of nucleic acid hybridization using zwitterionic compound**
Verfahren zur Erhöhung der Spezifität von Nukleinsäurehybridisierung unter Verwendung einer zwitterionischen Verbindung
Procédé d'augmentation de la spécificité de l'hybridation d'acide nucléique à l'aide d'un composé zwittérionique

(30) Priority: 07.04.2006 KR 20060031932
(43) Date of publication of application: 10.10.2007
(73) Proprietor: Samsung Electronics Co., Ltd., Suwon-si, Gyeonggi-Do (KR)
(72) Inventor: Lee, Jung-nam c/o Samsung Advanced Institute of Technology, Yongsin-si Gyeonggi-do (KR); Peak, Sang-hyun c/o Samsung Advanced Institute of Technology, Yongsin-si Gyeonggi-do (KR)
(74) Representative: Grünecker, Kinkeldey, Stockmair & Schwanhäusser Anwaltssozietät

(56) References cited:
- WO-A-98/10273
- WO-A-02/055985
- WO-A1-00/34521
- GB-A- 2 378 445
- US-A- 5 853 986
- US-A- 5 935 825
- US-A- 6 114 150
- "SIMULTANEOUS DETECTION OF MICROORGANISMS IN SOIL SUSPENSION BASED ON PCR AMPLIFICATION OF BACTERIAL 16S RRNA FRAGMENTS" BIOTECHNIQUES, INFORMA LIFE SCIENCES PUBLISHING, WESTBOROUGH, MA, US, vol. 21, no. 3, September 1996 (1996-09), pages 463-468, XP001247274 ISSN: 0736-6205

## Description

### BACKGROUND OF THE INVENTION

### 1. Field of the Invention

The present invention relates to a method of increasing the specificity of nucleic acid hybridization using specific zwitterionic compounds.

### 2. Description of the Related Art

Hybridization specificity (also known as hybridization stringency) refers to the extent to which complementary nucleic acids form specific hybrids with each other, when two complementary nucleic acids and a non-complementary nucleic acid undergo hybridization. In general, attempts have been made to obtain higher hybridization specificities by adjusting the salt concentration in the buffer and the reaction temperature, or by adding additives such as Denhart's solution, or adding a non-specific DNA or RNA to induce competition for hybridization.

Polymerase chain reaction (PCR) is a method of amplifying nucleic acids, comprising denaturation, annealing and extension of nucleic acids, and during the process of annealing, hybridization of a probe and a target nucleic acid takes place. The conditions for the PCR can be altered according to the degree of homology between the probe and the target nucleic acid. When the annealing temperature is raised under given PCR conditions, the yield of non-specific hybridization products is decreased, whereas, when the annealing temperature is lowered, the yield of non-specific hybridization products is increased. See, e.g. J. Sambrook and David W. Russell: Molecular Cloning: A Laboratory Manual, Cold Spring Harbor Laboratory Press, (2000).

The hybridization specificity between the primer nucleic acid and the target nucleic acid exerts a significant effect on a PCR, and particularly on a multiplex PCR. A multiplex PCR enables simultaneous amplification of different genes that are intended for amplification, in the same reaction. That is to say, different primer sets which are able to amplify their respective target sequences are placed in one reactor, and amplifications of the target sequences are simultaneously performed in a single PCR reaction (see e.g. Wittwer C.T., et al., Real-time multiplex PCR assays. Methods 25(4): 430-442, (2001); Markoulatos P., et al., Multiplex PCR: rapid DNA cycling in a conventional thermal cycler. J. Clin. Lab. Anal. 17(4): 108-112, (2003); O. Henegariu, et al., Multiplex PCR: Critical Parameters and Step-by-Step Protocol. BioTechniques 23: 504-511, (1997); and Markoulatos P., et al., Multiplex polymerase chain reaction: a practical approach. J. Clin. Lab. Anal. 16(1): 47-51, (2002). The primers for the multiplex PCR should be specific only to the target DNA sequences, and should not interfere with other primers' reactions, so that the target DNAs can be sufficiently amplified. A multiplex PCR results in many target hybridization/ amplification products that are indicated by a number of electrophoretic bands. If the hybridization specificity is low in the annealing process, numerous bands may appear on the electrophoresis gel in addition to the plurality of targeted bands, causing a problem in analyzing the multiplex PCR results. Therefore, increasing the hybridization specificity is strongly required.

US Patent No. 4,936,963 discloses a method of reducing the time taken by electrophoresis using histidine, which is a zwitterionic substance, in order to lower the overall conductivity in the electrophoresis. This invention makes use of a zwitterionic substance, as is done by the present invention. However, the object of the use of the zwitterionic substance is to reduce the time taken by electrophoresis, and is different from the object of the present invention which is to increase the nucleic acid hybridization specificity.

WO 02/055985 A discloses methods and compositions useful for identifying and diagnosing rare fetal cells in a mixed cell population, as well as kits useful for practicing said methods. The methods entail the use of specific nucleic acid probes that hybridize to fetal cell associated RNAs for fetal cell detection.

"Simultaneous Detection of Microorganisms in Soil Suspension Based on PCR Amplification of Bacterial 16S RRNA Fragments" Biotechniques, Informa Life Sciences Publishing, Westborough, MA, US, vol. 21, no. 3, September 1996 (1996-09), pages 463-468 refers to the simultaneous detection of microorganisms in soil suspension based on multiplex PCR amplification of bacterial 16S rRNA fragments. The effect of buffer compositions, namely Tris, AMPSO and CAPSO buffers on the amplification is investigated.

US-A-5,935,825 teaches a method for PCR amplification wherein PCR is carried out at a higher pH than the pH widely used in prior art processes, in particular a pH between 9 and 11.

In this regard, the inventors of the present invention conducted research in order to address the problems revealed by the existing technologies of the related art, and found that an increase in the specificity of nucleic acid hybridization can be induced by adding specific zwitterionic compounds to a nucleic acid hybridization reaction, especially a multiplex PCR.

### SUMMARY OF THE INVENTION

The present invention provides a method of increasing the specificity of nucleic acid hybridization, the method comprising hybridizing nucleic acids in a solution containing a zwitterionic compound selected from the group consisting of 3-(cyclohexylamino)-1-propanesulfonic acid (CAPS), 3-(cyclohexylamino)-2-hydroxy-1-propane sulfonate (CAPSO), and 2-(cyclohexylamino)ethane sulfonate (CHES), wherein the concentration of CAPS in the solution is 1 to 3% by weight, wherein the concentration of CAPSO in the solution is 0.8 to 3% by weight, and/or wherein the concentration of CHES in the solution is 0.8 to 3% by weight.

According to another aspect of the present invention, there is provided a method of amplifying a target nucleic acid, the method comprising hybridizing a primer and the target nucleic acid in a solution containing a zwitterionic compound selected from the group consisting of 3-(cyclohexylamino)-1-propanesulfonic acid (CAPS), 3-(cyclohexylamino)-2-hydroxy-1-propane sulfonate (CAPSO), and 2-(cyclohexylamino)ethane sulfonate (CHES) in the course of an amplification reaction, such as, for example, a PCR, wherein the concentration of CAPS in the solution is 1 to 3% by weight, wherein the concentration of CAPSO in the solution is 0.8 to 3% by weight; and/or wherein the concentration of CHES in the solution is 0.8 to 3% by weight.

According to another aspect of the present invention, there is provided a method of detecting a pathogen by amplifying a target nucleic acid related to a pathogen, the method comprising
hybridizing a primer and the target nucleic acid in a solution containing a zwitterionic compound selected from the group consisting of
3-(cyclohexylamino)-1-propanesulfonic acid (CAPS),
3-(cyclohexylamino)-2-hydroxy-1-propane sulfonate (CAPSO), and
2-(cyclohexylamino)ethane sulfonate (CHES);
and amplification of the target nucleic acid using a multiplex polymerase chain reaction (PCR), wherein the pathogen is a virus which causes a respiratory disease in human beings, and wherein the concentration of CAPS in the solution is 1 to 3% by weight, and wherein the concentration of CAPSO in the solution is 0.8 to 3% by weight, and/or wherein the concentration of CHES in the solution is 0.8 to 3% by weight.

### BRIEF DESCRIPTION OF THE DRAWINGS

The above and other features and advantages of the present invention will become more apparent by describing in detail exemplary embodiments thereof with reference to the attached drawings in which:
FIG. 1 is a photographic image showing the results of an electrophoretic analysis of PCR products obtained when 3-(cyclohexylamino)-1-propanesulfonic acid (CAPS) was added to a multiplex PCR, the results showing a decrease in the yield of non-specific hybridization products;
FIG. 2 is a spectrum obtained using an Agilent 2100 Bioanalyzer in a quantitative evaluation of the yields of target hybridization products and non-specific hybridization products;
FIG. 3 is a photographic image showing the results of an electrophoretic analysis of PCR products obtained when 3-(cyclohexylamino)-2-hydroxy-1-propane sulfonate (CAPSO) was added to a multiplex PCR, the results showing a decrease in the yield of non-specific hybridization products;
FIG. 4 is a photographic image showing the results of an electrophoretic analysis of PCR products obtained when 2-(cyclohexylamino)ethane sulfonate (CHES) was added to a multiplex PCR, the results showing a decrease in the yield of non-specific hybridization products; and
FIG. 5 is a photographic image showing the results of an electrophoretic analysis of PCR products obtained when betaine was added to a multiplex PCR, the results showing a decrease in the yield of non-specific hybridization products.

### DETAILED DESCRIPTION OF THE INVENTION

The present invention will now be described more fully with reference to the accompanying drawings, in which exemplary embodiments of the invention are shown. The invention may, however, be embodied in many different forms and should not be construed as being limited to the embodiments set forth herein; rather, these embodiments are provided so that this disclosure will be thorough and complete, and will fully convey the concept of the invention to those skilled in the art.

Hybridization of nucleic acids refers to the binding between nucleic acids of different origins, and the conditions for hybridization can be varied according to the sequence homology of the nucleic acids to be bound. In other words, if the sequence homology between the subject nucleic acids is high, stringent conditions are used, and if the sequence homology is low, mild conditions are used. When the hybridization conditions are stringent, the hybridization specificity increases, and this increase of the hybridization specificity leads to a decrease in the yield of non-specific hybridization products. However, under mild hybridization conditions, the hybridization specificity decreases, and this decrease in the hybridization specificity leads to an increase in the yield of non-specific hybridization products. Particularly in the case of the latter, there is a need to reduce the level of the non-specific hybridization products, and therefore, an attempt was made in the present invention to increase the nucleic acid hybridization specificity by hybridizing nucleic acids in a solution containing a specific zwitterionic compound.

Hybridization between nucleic acids can be classified into hybridization between a DNA and a DNA, hybridization between a DNA and an RNA, and hybridization between an RNA and an RNA. Nucleic acid hybridization reactions are usually classified into Southern hybridization reactions or Northern hybridization reactions. Nucleic acid hybridization reactions are particularly important for DNA chips, where a rapid detection of a target nucleic acid which is specific to a probe is effected by a hybridization reaction, in which the probe is immobilized on a chip, and a sample containing the target nucleic acid is added to the immobilized probe.

A zwitterionic compound refers to a compound capable of acting simultaneously as an acid as well as a base in a neutral solution. Examples of zwitterionic compounds include betaine, amino acids, 3-(cyclohexylamino)-1-propanesulfonic acid (CAPS), 3-[(3-cholamidopropyl)dimethylammonio]-1-propane sulfonate (CHAPS), 3-(cyclohexylamino)-2-hydroxy-1-propane sulfonate (CAPSO), 2-(cyclohexylamino)ethane sulfonate (CHES), and the like. The zwitterionic compounds, betaine and CAPS, have structures as shown below.

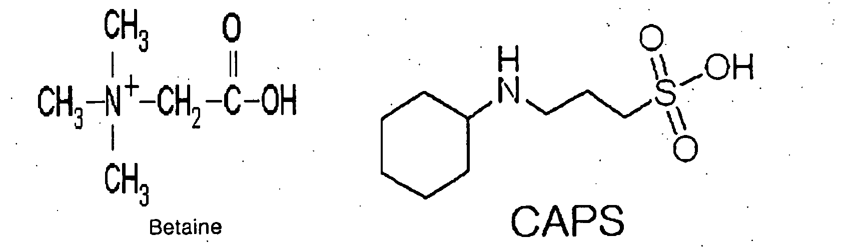

Zwitterionic compounds in most cases tend to increase the nucleic acid hybridization specificity in a nucleic acid hybridization reaction, thus decreasing the yield of non-specific hybridization products, but unfortunately, the same compounds also decrease the yield of target hybridization products. However, some zwitterionic compounds show desirable characteristics of decreasing the yield of non-specific hybridization products to increase the nucleic acid hybridization specificity, while maintaining the yield of target hybridization products. The inventors of the present invention discovered zwitterionic compounds having such desirable characteristics, including 3-(cyclohexylamino)-1-propanesulfonic acid (CAPS), 3-[(3-cholamidopropyl)dimethylammonio]-1-propane sulfonate (CHAPS), 3-(cyclohexylamino)-2-hydroxy-1-propane sulfonate (CAPSO), 2-(cyclohexylamino)ethane sulfonate (CHES), and the like.

When such a zwitterionic compound according to an embodiment of the present invention is added to a nucleic acid hybridization reaction, the specificity of the nucleic acid hybridization reaction is increased, and as a result, generation of false positive bands, which present a significant problem during the hybridization reaction, can be reduced. The false positive bands are generated to an increasing extent when the specificity of nucleic acid hybridization reaction decreases. These false positive bands interfere with the efficient electrophoretic separation of target genes, and adversely affect the analysis process in terms of processing time and operating costs. Accordingly, elimination of such non-specific hybridization products is strongly desired in the separation of target genes.

When the concentration of the zwitterionic compound is lower than 0.2% by weight, the nucleic acid hybridization specificity is decreased, and the yield of non-specific hybridization products will accordingly increase. When the concentration of the zwitterionic compound is higher than 3% by weight, the yield of target hybridization products is decreased.

According to another embodiment of the present invention, the method of amplifying a target nucleic acid comprises denaturation, annealing and extension, wherein a hybridization between a probe and a target nucleic acid occurs during the process of annealing. When the zwitterionic compound according to an embodiment of the present invention is added to the hybridization reaction, the specificity of the hybridization between the probe and the target nucleic acid is increased. This increase in the nucleic acid hybridization specificity leads to a decrease in the yield of non-specific amplification products, and only the desired target hybridization products are specifically amplified.

Examples of the method of amplifying a target nucleic acid include polymerase chain reaction (PCR), ligase chain reaction, stranded-displacement amplification, nucleic acid-based amplification, repair chain reaction, helicase chain reaction, QB replicase amplification, and ligation activated transcription. Preferably, the amplification of a target nucleic acid is performed by polymerase chain reaction.

According to an embodiment of the present invention, the PCR is a multiplex PCR. The method of amplifying a target nucleic acid according to the current embodiment of the present invention is intended to increase the specificity of a nucleic acid hybridization reaction, and more specifically, is intended to eliminate non-specific hybridization products of the multiplex PCR during the amplification of a target gene, which is useful for identification of a plurality of pathogens [see e.g. Elnifro E.M., et al., Multiplex PCR: optimization and application in diagnostic virology. Clin. Microbiol. Rev. 13(4): 559-570, (2000)]. Zwitterionic compounds in most cases tend to increase the nucleic acid hybridization specificity in a nucleic acid hybridization reaction, thus decreasing the yield of non-specific products of the hybridization, but unfortunately, the same compounds also decrease the yield of target hybridization products. However, some zwitterionic compounds show desirable characteristics of decreasing the yield of non-specific hybridization products to increase the nucleic acid hybridization specificity, while maintaining the yield of target hybridization products. These desirable characteristics constitute an essential factor that enables accurate identification of specific pathogens by decreasing false positive bands in the multiplex PCR.

The production of non-specific hybridization products is a particular problem in a multiplex PCR. A multiplex PCR allows to simultaneously detect a number of genes through a single PCR, and a plurality of bands appear on an electrophoresis gel. If too many bands of non-specific hybridization products are generated in addition to the bands of target gene hybridization products of desired sizes in a multiplex PCR, it is impossible to perform an accurate identification of specific pathogens. Accordingly, it is necessary to reduce the number of non-specific hybridization products in the multiplex PCR. When an appropriate amount of the zwitterionic compound according to an embodiment of the present invention is added to the multiplex PCR, the number of non-specific hybridization products is significantly decreased.

According to another embodiment of the present invention, the levels of non-specific hybridization products are minimized in the multiplex PCR, while the level of the desired target hybridization products is maintained. As discussed above, when an appropriate amount of the zwitterionic compound is added to the multiplex PCR, the number of non-specific hybridization products is significantly decreased. However, if the yield of desired target hybridization products is also decreased along with the decrease in the non-specific hybridization products, the desired effect cannot be achieved. Zwitterionic compounds in most cases simultaneously decrease the yield of non-specific hybridization products and the yield of target hybridization products. However, when the zwitterionic compound according to an embodiment of the present invention is used, a reduction in the yield of non-specific hybridization products is achieved while the yield of desired target hybridization products is maintained.

When the concentration of the zwitterionic compound is lower than 0.2% by weight, the nucleic acid hybridization specificity is decreased, and the yield of non-specific hybridization products will be increased. When the concentration of the zwitterionic compound is higher than 3% by weight, the yield of target hybridization products will be decreased.

According to another embodiment of the present invention, the primer is specific to the detection of a plurality of pathogens. When the multiplex PCR is performed using the zwitterionic compound according to an embodiment of the present invention, the number of non-specific hybridization products is decreased, while the yield of target hybridization products is maintained. Thus, it is now possible to easily solve the problem of the prior art that accurate detection of a plurality of genes is difficult due to the high yield of non-specific hybridization products. The detection of a plurality of genes encompasses the simultaneous analysis of nucleic acid sequences that are related to pathogens, genetic diseases and the like. For example, the most common viruses which cause respiratory diseases in human beings include measles virus, enterovirus, rhinovirus

SARS-associated coronavirus (SARS-coV), varicella zoster virus (VSV), adenovirus, human parainfluenza virus 1 (HPIV 1), human parainfluenza virus 2 (HPIV 2), human parainfluenza virus 3 (HPIV 3), influenza virus A (IVA), influenza virus B (IVB), respiratory syncytial virus A (RSVA), and respiratory syncytial virus B (RSVB). Rapid and specific detection of these viruses is essential to the diagnosis, prevention and treatment of respiratory diseases, and the viruses can be detected rapidly and accurately by performing the multiplex PCR using the zwitterionic compound according to an embodiment of the present invention.

The solution for nucleic acid hybridization according to the current embodiment of the present invention contains various compounds needed in a nucleic acid hybridization reaction [see e.g. J. Sambrook and David W. Russell: Molecular Cloning: A Laboratory Manual, Cold Spring Harbor Laboratory Press, (2000)]. When a nucleic acid hybridization reaction is performed using the solution for nucleic acid hybridization according to the current embodiment, which contains the zwitterionic compound according to an embodiment of the present invention in addition to the various compounds, the specificity of the nucleic acid hybridization is increased, and better hybridization results can be obtained. The nucleic acid hybridization reaction is particularly important for DNA chips. When a probe is immobilized on a chip, a sample containing a target nucleic acid is added thereto, and a nucleic acid hybridization reaction is performed using the solution for nucleic acid hybridization of the present embodiment, and the target nucleic acid which is specific to the probe can be rapidly detected.

Hereinafter, the present invention will be described in more detail with reference to Examples. These Examples are for illustrative purposes only, and should not be construed to limit the scope of the present invention by any means.

### EXAMPLE 1: Effect of CAPS on the specificity of nucleic acid hybridization in a multiplex PCR

As a template for a multiplex PCR, genomic DNA (gDNA) was isolated from Haemophilus influenza, which is a main pathogen for respiratory infections, , and the isolated gDNA was used in an amount of 0.2 ng or 1 ng in a multiplex PCR. 3-(cyclohexylamino)-1-propanesulfonic acid (CAPS) was used as the zwitterionic compound according to an embodiment of the present invention at a concentration of 0.5% or 1% to perform a multiplex PCR. The multiplex PCR was performed using a GeneAmp PCR system 9700 (ABI), with a PCR mixture (template gDNA 0.2 ng or 1 ng, CAPS 0.5% or 1%,1×Taq Pol buffer, 200 pM each of dNTP mixture, 400 nM each of PCR primer (SEQ ID NO:1 through SEQ ID NO:10), and 5 units of Taq Polymerase) at 95°C for 1 minute, by completely denaturing the DNA, then subjecting the PCR mixture to the PCR for 25 cycles (5 sec at 95°C, 13 seconds at 62°C, and 15 seconds at 72°C), and conducting the final extension at 72°C for 1 minute.

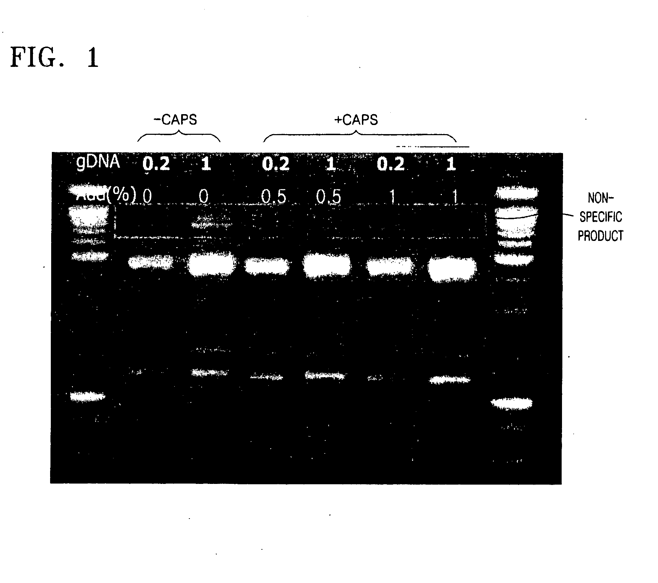

FIG. 1 is a photographic image showing the results of an electrophoretic analysis of PCR products obtained when CAPS was added to a multiplex PCR, the results showing a decrease in the yield of non-specific hybridization products. Referring to FIG. 1, it can be seen that in the samples having CAPS added (indicated as +CAPS), the intensity of the bands of non-specific hybridization products significantly decreases, as compared with the samples not having CAPS added (indicated as - CAPS), and the intensity of the bands of non-specific hybridization products markedly decreases as the concentration of CAPS increases. In the case of having CAPS added (+CAPS), the intensity of the bands of non-specific hybridization products markedly decreases, while the intensity of the target hybridization product bands remains constant. Furthermore, as the amount of the template DNA increases as indicated, the amount of the desired target hybridization products and the amount of the non-specific hybridization products also increase.

During the treatment with CAPS, in order to quantitatively evaluate the extent of production of target hybridization products and non-specific hybridization products, each of the PCR products was subjected to electrophoresis, and then fluorescence was detected using an Agilent 2100 Bioanalyzer.

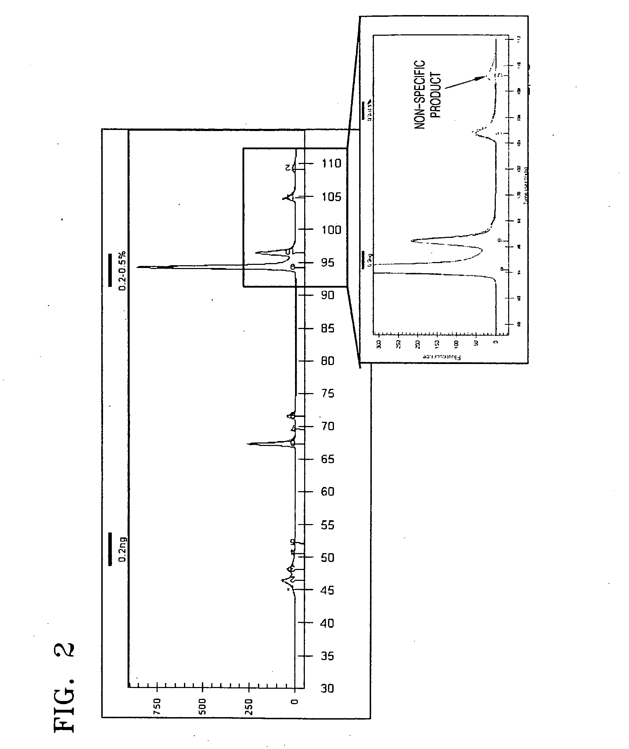

FIG. 2 is a spectrum obtained using an Agilent 2100 Bioanalyzer of a quantitative evaluation of the yields of target hybridization products and non-specific hybridization products. Referring to FIG. 2, among the two curves shown in the magnified graph below the main graph of FIG. 2, the upper curve corresponds to the PCR sample with 0.2 ng of the gDNA and without CAPS, while the lower curve corresponds to the PCR sample with 0.2 ng of the gDNA with 0.5% CAPS. The band positions for the non-specific hybridization products are indicated with arrows. As shown in FIG. 2, the intensities of the target hybridization product bands were nearly consistent, regardless of whether the sample contained CAPS or not. However, the bands of the non-specific hybridization products were mostly eliminated when the PCR reaction was performed with CAPS added to the sample.

Therefore, it can be seen that when CAPS, a zwitterionic compound according to an embodiment of the present invention, is added to the multiplex PCR, the yield of non-specific hybridization products is significantly decreased, while the yield of the target hybridization products remains constant. Thus, addition of CAPS is found to be useful for detection of specific pathogens.

### EXAMPLE 2: Effect of CAPSO and CHES on the nucleic acid hybridization specificity in multiplex PCR

To investigate the nucleic acid hybridization specificity of different types of the zwitterionic compound in the multiplex PCR, CAPSO and CHES were used as additives. The experiment was performed in the same manner as in Example 1, except that CAPSO and CHES were added to the multiplex PCR respectively at concentrations of 0.2%, 0.4%, 0.8% and 1.6%, and 1 ng of the Haemophilus influenza gDNA template was used.

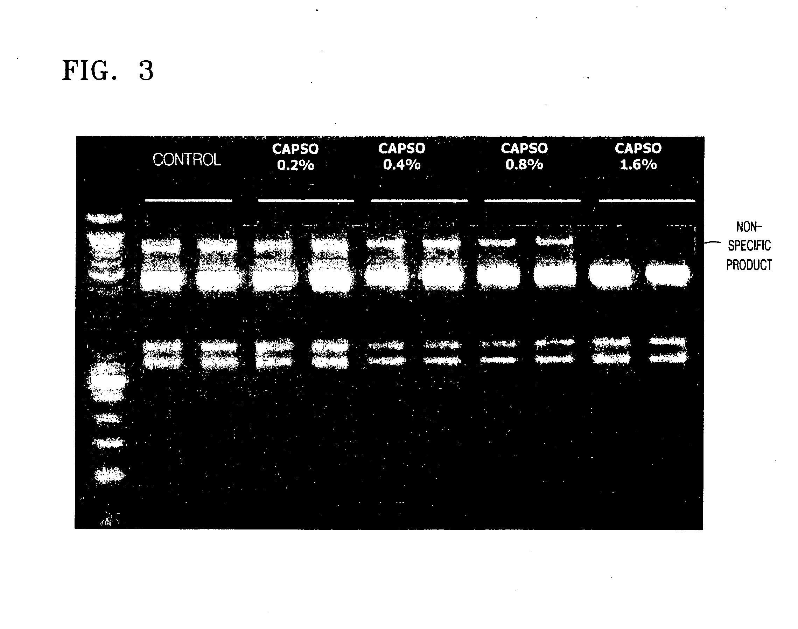

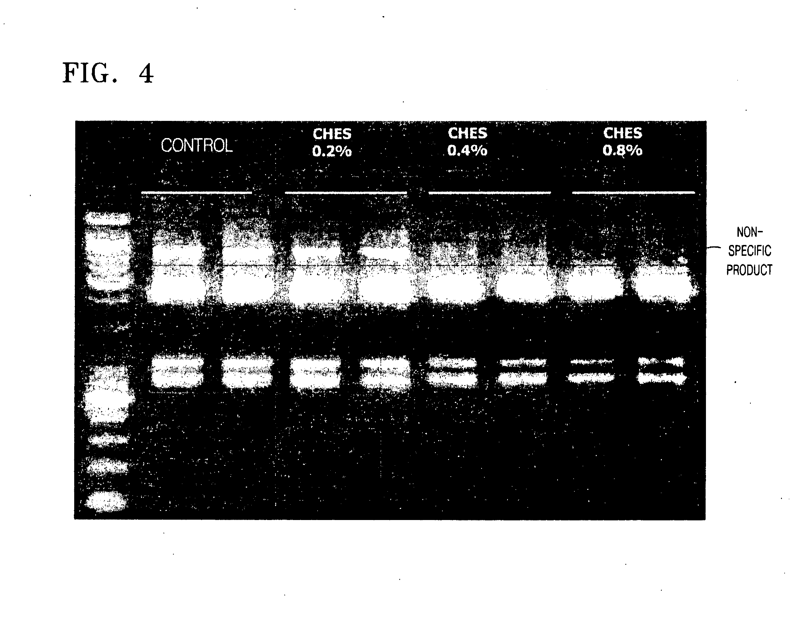

FIG. 3 is a photographic image showing the results of an electrophoretic analysis of PCR products obtained when CAPSO was added to a multiplex PCR, the results showing a decrease in the yield of non-specific hybridization products. Referring to FIG. 3, the control was subjected to a multiplex PCR without adding CAPSO. As can be seen in FIG. 3, the intensity of the band of non-specific hybridization products markedly decreased in the samples with CAPSO as compared with the samples without CAPSO, and a higher concentration of CAPSO resulted in a more noticeable decrease in the band intensity of exclusively the non-specific band. FIG. 4 is a photographic image showing the results of an electrophoretic analysis of PCR products obtained when CHES was added to a multiplex PCR, the results showing a decrease in the yield of non-specific hybridization products. As shown in FIG. 4, CHES yielded similar results to those of CAPSO.

### EXAMPLE 3: Effect of the type of zwitterionic compound on nucleic acid hybridization specificity in a multiplex PCR

Betaine was used in investigating the nucleic acid hybridization specificity of different types of the zwitterionic compound in a multiplex PCR. The same experiment was performed in the same manner as in Example 1, except that betaine was added to a multiplex PCR using 5xband doctor of Solgent, Inc., in amounts of 2.5 µl (0.25×), 5 µl (0.5×), 10 µl (1×), and 15 µl (1.5×), and the Haemophilus influenza gDNA template was used in amounts of 1 ng, 0.1 ng and 0.01 ng, respectively.

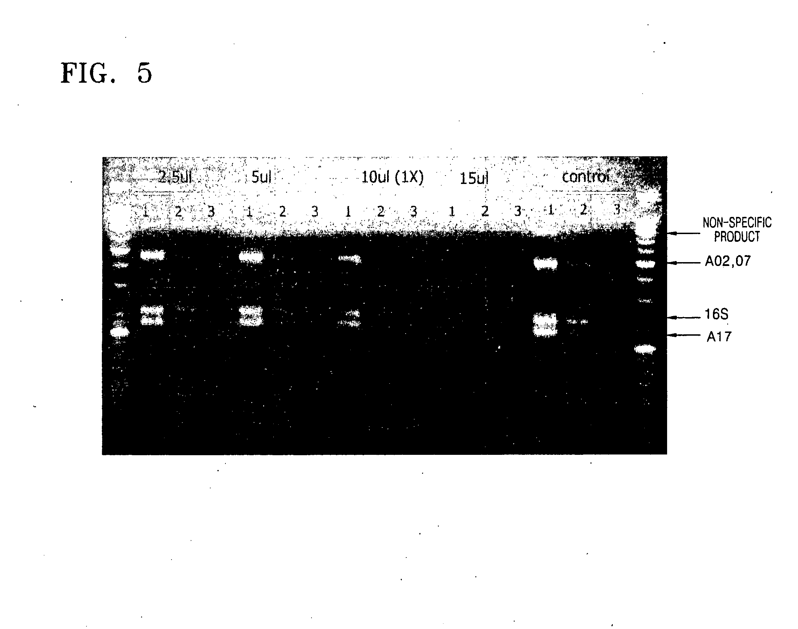

FIG. 5 is a photographic image showing the results of an electrophoretic analysis of PCR products obtained when betaine was added to a multiplex PCR, the results showing a decrease in the yield of non-specific hybridization products. Referring to FIG. 5, the control was subjected to the multiplex PCR without adding betaine, and the numerals "1 ", "2" and "3" refer to the amounts of the template gDNA used in the PCR, such as 1 ng, 0.1 ng and 0.01 ng, respectively. As can be seen in FIG. 5, the intensity of the band of non-specific hybridization products was markedly decreased in the case of adding betaine as compared with the case of not adding betaine, and a higher concentration of betaine resulted in a more noticeable decrease in the band intensity. However, it can be seen that unlike the case of CAPS, although the intensity of the non-specific hybridization product band markedly decreases with an increasing concentration of betaine, the intensities of the bands of desired target hybridization products also markedly decrease. Therefore, betaine is not suitable for the purpose of increasing the nucleic acid hybridization specificity to detect specific pathogens.

Therefore, not all zwitterionic compounds show the effect of the present invention, and only specific zwitterionic compounds such as CAPS can result in a decrease of non-specific hybridization products, while maintaining the yield of desired target hybridization products.

As discussed above, the method according to embodiments of the present invention allows reduction in the yield of non-specific hybridization products while maintaining the yield of target hybridization products in a multiplex PCR, thus increasing the specificity of nucleic acid hybridization, and can be effectively used in the identification of specific pathogens, diagnosis of genetic diseases, analysis of gene sequences, and the like.

While the present invention has been particularly shown and described with reference to exemplary embodiments thereof, it will be understood by those of ordinary skill in the art that various changes in form and details may be made therein without departing from the scope of the present invention as defined by the following claims.
## Claims

1. A method of increasing the specificity of nucleic acid hybridization, the method comprising
hybridizing nucleic acid in a solution containing a zwitterionic compound selected from the group consisting of 3-(cyclohexylamino)-1-propanesulfonic acid (CAPS),
3-(cyclohexylamino)-2-hydroxy-1-propane sulfonate (CAPSO), and
2-(cyclohexylamino)ethane sulfonate (CHES), wherein the concentration of CAPS in
the solution is 1 to 3% by weight, wherein the concentration of CAPSO in the solution is 0.8 to 3% by weight, and/or wherein the concentration of CHES in the solution is 0.8 to 3% by weight.

2. The method of claim 1, wherein the hybridization is selected from the group consisting of hybridization between a DNA and a DNA, hybridization between a DNA and an RNA, and hybridization between an RNA and an RNA.

3. A method of amplifying a target nucleic acid, the method comprising hybridizing a primer and the target nucleic acid in a solution containing a zwitterionic compound selected from the group consisting of
3-(cyclohexylamino)-1-propanesulfonic acid (CAPS),
3-(cyclohexylamino)-2-hydroxy-1-propane sulfonate (CAPSO), and
2-(cyclohexylamino)ethane sulfonate (CHES);
and amplification of the target nucleic acid, wherein the concentration of CAPS in the solution is 1 to 3% by weight, wherein the concentration of CAPSO in the solution is 0.8 to 3% by weight, and/or wherein the concentration of CHES in the solution is 0.8 to 3% by weight.

4. The method of claim 3, wherein the amplification of the target nucleic acid is performed using a polymerase chain reaction (PCR).

5. The method of claim 4, wherein the polymerase chain reaction is a multiplex PCR.

6. The method of claim 3, wherein the primer is specific to the detection of a plurality of pathogens.

7. A method of detecting a pathogen by amplifying a target nucleic acid related to a pathogen, the method comprising
hybridizing a primer and the target nucleic acid in a solution containing a zwitterionic compound selected from the group consisting of
3-(cyclohexylamino)-1-propanesulfonic acid (CAPS),
3-(cyclohexylamino)-2-hydroxy-1-propane sulfonate (CAPSO), and
2-(cyclohexylamino)ethane sulfonate (CHES);
and amplification of the target nucleic acid using a multiplex polymerase chain reaction (PCR), wherein the pathogen is a virus which causes a respiratory disease in human beings, and wherein the concentration of CAPS in the solution is 1 to 3% by weight, and wherein the concentration of CAPSO in the solution is 0.8 to 3% by weight, and/or wherein the concentration of CHES in the solution is 0.8 to 3% by weight.

8. The method of claim 7, wherein the primer is specific to the detection of a plurality of pathogens.

9. The method of claim 7 or 8, wherein the virus is at least one of measles virus, enterovirus, rhinovirus, SARS-associated coronavirus (SARS-coV), varicella zoster virus (VSV), adenovirus, human parainfluenza virus (HPIV 1), human parainfluenza virus 2 (HPIV 2), human parainfluenza virus 3 (HPIV 3), influenza virus A (IVA), influenza virus B (IVB), respiratory syncytial virus A (RSVA), and respiratory syncytial virus B (RSVB).

## Patentansprüche

1. Verfahren zum Erhöhen der Spezifität einer Nukleinsäurehybridisierung, wobei das Verfahren umfasst
das Hybridisieren von Nukleinsäure in einer Lösung, die eine zwitterionische Verbindung enthält, die ausgewählt ist aus der Gruppe bestehend aus 3-(Cyclohexylamino)-1-propansulfonsäure (CAPS), 3-(Cyclohexylamino)-2-hydroxy-1-propan-sulfonat (CAPSO), und 2-(Cyclohexylamino)ethan-sulfonat (CHES), wobei die Konzentration von CAPS in der Lösung 1 bis 3 Gew.-% beträgt, wobei die Konzentration von CAPSO in der Lösung 0,8 bis 3 Gew.-% beträgt und/oder wobei die Konzentration von CHES in der Lösung 0,8 bis 3 Gew.-% beträgt.

2. Verfahren nach Anspruch 1, wobei die Hybridisierung ausgewählt ist aus der Gruppe bestehend aus einer Hybridisierung zwischen einer DNA und einer DNA, einer Hybridisierung zwischen einer DNA und einer RNA und einer Hybridisierung zwischen einer RNA und einer RNA.

3. Verfahren zum Amplifizieren einer Zielnukleinsäure, wobei das Verfahren umfasst das Hybridisieren eines Primers und der Zielnukleinsäure in einer Lösung, die eine zwitterionische Verbindung enthält, die ausgewählt ist aus der Gruppe bestehend aus 3-(Cyclohexylamino)-1-propansulfonsäure (CAPS), 3-(Cyclohexylamino)-2-hydroxy-1-propan-sulfonat (CAPSO), und 2-(Cyclohexylamino)ethan-sulfonat (CHES);
und die Amplifikation der Zielnukleinsäure, wobei die Konzentration von CAPS in der Lösung 1 bis 3 Gew.-% beträgt, wobei die Konzentration von CAPSO in der Lösung 0,8 bis 3 Gew.-% beträgt und/oder wobei die Konzentration von CHES in der Lösung 0,8 bis 3 Gew.-% beträgt.

4. Verfahren nach Anspruch 3, wobei die Amplifikation der Zielnukleinsäure unter Verwendung einer Polymerase-Kettenreaktion (PCR) durchgeführt wird.

5. Verfahren nach Anspruch 4, wobei die Polymerase-Kettenreaktion eine Multiplex-PCR ist.

6. Verfahren nach Anspruch 3, wobei der Primer für den Nachweis einer Mehrzahl von Pathogenen spezifisch ist.

7. Verfahren zum Nachweisen eines Pathogens durch Amplifizieren einer Zielnukleinsäure, die einem Pathogen zugeordnet ist, wobei das Verfahren umfasst
das Hybridisieren eines Primers und der Zielnukleinsäure in einer Lösung, die eine zwitterionische Verbindung enthält, die ausgewählt ist aus der Gruppe bestehend aus 3-(Cyclohexylamino)-1-propansulfonsäure (CAPS), 3-(Cyclohexylamino)-2-hydroxy-1-propan-sulfonat (CAPSO), und 2-(Cyclohexylamino)ethan-sulfonat (CHES);
und die Amplifikation der Zielnukleinsäure unter Verwendung einer Multiplex-Polymerase-Kettenreaktion (PCR), wobei das Pathogen ein Virus ist, welches eine Atemwegserkrankung in Menschen verursacht, und wobei die Konzentration von CAPS in der Lösung 1 bis 3 Gew.-% beträgt und wobei die Konzentration von CAPSO in der Lösung 0,8 bis 3 Gew.-% beträgt und/oder wobei die Konzentration von CHES in der Lösung 0,8 bis 3 Gew.-% beträgt.

8. Verfahren nach Anspruch 7, wobei der Primer für den Nachweis einer Mehrzahl von Pathogenen spezifisch ist.

9. Verfahren nach Anspruch 7 oder 8, wobei das Virus wenigstens eines der folgenden Viren ist: Masernvirus, Enterovirus, Rhinovirus, SARS-assoziiertes Coronavirus (SARS-coV), Varizella-Zoster-Virus (VSV), Adenovirus, humanes Parainfluenzavirus (HPIV 1), humanes Parainfluenzavirus 2 (HPIV 2), humanes Parainfluenzavirus 3 (HPIV 3), Influenzavirus A (IVA), Influenzavirus B (IVB), respiratorisches Synzytialvirus A (RSVA) und respiratorisches Synzytialvirus B (RSVB).

## Revendications

1. Procédé pour augmenter la spécificité d'hybridation d'un acide nucléique, ledit procédé consistant à
hybrider un acide nucléique dans une solution contenant un composé zwittérionique choisi dans le groupe constitué de l'acide 3-(cyclohexylamino)-1-propanesulfonique (CAPS),
du 3-(cyclohexylamino)-2-hydroxy-1-propanesulfonate (CAPSO), et
du 2-(cyclohexyl)aminoéthanesulfonate (CHES), la concentration du CAPS dans la solution étant de 1 à 3 % en poids, la concentration du CAPSO dans la solution étant de 0,8 à 3 % en poids, et/ou la concentration du CHES dans la solution étant de 0,8 à 3 % en poids.

2. Procédé selon la revendication 1, dans lequel l'hybridation est choisie dans le groupe constitué d'une hybridation entre un ADN et un ADN, d'une hybridation entre un ADN et un ARN, et d'une hybridation entre un ARN et un ARN.

3. Procédé d'amplification d'un acide nucléique cible, ledit procédé consistant à
hybrider une amorce et l'acide nucléique cible dans une solution contenant un composé zwittérionique choisi dans le groupe constitué de
l'acide 3-(cyclohexylamino)-1-propanesulfonique (CAPS),
le 3-(cyclohexylamino)-2-hydroxy-1-propanesulfonate (CAPSO), et
le 2-(cyclohexylamino)éthanesulfonate (CHES) ;
et amplifier l'acide nucléique cible, la concentration du CAPS dans la solution étant de 1 à 3 % en poids, la concentration du CAPSO dans la solution étant de 0,8 à 3 % en poids, et/ou la concentration du CHES dans la solution étant de 0,8 à 3 % en poids.

4. Procédé selon la revendication 3 dans lequel l'amplification de l'acide nucléique cible est effectuée au moyen d'une réaction en chaîne de la polymérase (PCR).

5. Procédé selon la revendication 4, dans lequel la réaction en chaîne de la polymérase est une PCR multiplex.

6. Procédé selon la revendication 3, dans lequel l'amorce est spécifique de la détection d'une pluralité d'agents pathogènes.

7. Procédé de détection d'un agent pathogène par amplification d'un acide nucléique cible en rapport avec un agent pathogène, ledit procédé consistant à
hybrider une amorce et l'acide nucléique cible dans une solution contenant un composé zwittérionique choisi dans le groupe constitué de
l'acide 3-(cyclohexylamino)-1-propanesulfonique (CAPS),
le 3-(cyclohexylamino)-2-hydroxy-1-propanesulfonate (CAPSO), et
le 2-(cyclohexylamino)éthanesulfonate (CHES) ;
et amplifier l'acide nucléique cible par une réaction en chaîne de la polymérase (PCR) multiplex, l'agent pathogène étant un virus qui provoque une maladie respiratoire chez l'être humain, et la concentration du CAPS dans la solution étant de 1 à 3 % en poids, et la concentration du CAPSO dans la solution étant de 0,8 à 3 % en poids, et/ou la concentration du CHES dans la solution étant de 0,8 à 3 % en poids.

8. Procédé selon la revendication 7, dans lequel l'amorce est spécifique de la détection d'une pluralité d'agents pathogènes.

9. Procédé selon la revendication 7 ou 8, dans lequel le virus est au moins un des virus suivants : virus de la rougeole, entérovirus, rhinovirus, coronavirus associé au SRAS (syndrome respiratoire aigu sévère) (SRAS-coV), virus varicelle-zona (VZV), adénovirus, virus parainfluenza humain 1 (VPIH 1), virus parainfluenza humain 2 (VPIH 2), virus parainfluenza humain 3 (VPIH 3), virus grippal A, virus grippal B, virus respiratoire syncytial A (VRSA), et virus respiratoire syncytial B (VRSB).


<110> Samsung Electronics Co., Ltd.
<120> Method of increasing specificity of nucleic acid hybridization using zwitterionic compound
<130> EP45843FZ163pau
<140> not yet assigned <141> herewith
<150> KR 10-2005-0031932
   <151> 2006-04-07
<160> 10
<170> KopatentIn 1.71
<210> 1
   <211> 23
   <212> DNA
   <213> Artificial Sequence
<220>
   <223> forward primer
<400> 1
   gcgtaccttt tgtataatgg gtc 23
<210> 2
   <211> 19
   <212> DNA
   <213> Artificial Sequence
<220>
   <223> reverse primer
<400> 2
   gaccttagct ggcggtctg 19
<210> 3
   <211> 19
   <212> DNA
   <213> Artificial Sequence
<220>
   <223> forward primer
<400> 3
   tgtcgggtaa gttccgacc 19
<210> 4
   <211> 19
   <212> DNA
   <213> Artificial Sequence
<220>
   <223> reverse primer
<400> 4
   crgaaccacc ggatcacta 19
<210> 5
   <211> 20
   <212> DNA
   <213> Artificial Sequence
<220>
   <223> forward primer
<400> .5
   cacctcgatg tcgrctcatc 20
<210> 6
   <211> 21
   <212> DNA
   <213> Artificial sequence
<220>
   <223> reverse primer
<400> 6
   ggtcctctcg tactagrarc a 21
<210> 7
   <211> 22
   <212> DNA
   <213> Artificial sequence
<220>
   <223> forward primer
<400> 7
   tagcatatca gaaggcacac cc 22
<210> 8
   <211> 23
   <212> DNA
   <213> Artificial sequence
<220>
   <223> reverse primer
<400> 8
   atccactcaa gagagacaac att 23
<210> 9
   <211> 20
   <212> DNA
   <213> Artificial sequence
<220>
   <223> forward primer
<400> 9
   yccakactcc tacgggaggc 20
<210> 10
   <211> 22
   <212> DNA
   <213> Artificial sequence
<220>
   <223> reverse primer
<400> 10
   gtattaccgc rrctgctggc ac 22